# FASCICULE DE BREVET EUROPEEN

(11) **EP 0 953 346 B1**
(45) Date de publication et mention de la délivrance du brevet: **26.11.2003**
(21) Numéro de dépôt: 99400862.1
(22) Date de dépôt: 08.04.1999
(51) Int. Cl.: A61K 7/48, A61K 31/05

(54) **Utilisation d'au moins un hydroxystilbène dans une composition raffermissante**
Verwendung mindestens eines Hydroxystilben in einer straffenden Zusammensetzung
Use of at least a hydroxystilbene in a skin fortifying composition

(30) Priorité: 10.04.1998 FR 9804571
(43) Date de publication de la demande: 03.11.1999
(73) Titulaire: L'OREAL, 75008 Paris (FR)
(72) Inventeur: Breton, Lionel, 78000 Versailles (FR); Liviero, Christel, 75008 Paris (FR); Fagot, Dominique, 75020 Paris (FR)
(74) Mandataire: Tezier Herman, Béatrice

(56) Documents cités:
- PATENT ABSTRACTS OF JAPAN vol. 096, no. 011, 29 novembre 1996 (1996-11-29) & JP 08 175960 A (KAO CORP), 9 juillet 1996 (1996-07-09)
- DATABASE WPI Week 1098 Derwent Publications Ltd., London, GB; AN 98-105073 XP002095188 & JP 09 328410 A (MITSUBA BOEKI KK), 22 décembre 1997 (1997-12-22)

## Description

L'invention se rapporte à l'utilisation d'au moins un hydroxystilbène dans ou pour la préparation d'une composition raffermissante. En particulier, les compositions de l'invention sont destinées à stimuler la restructuration de la peau et/ou des muqueuses par la stimulation de la synthèse du collagène et/ou la stimulation de la prolifération des fibroblastes du derme.
L'invention a également pour objet une composition raffermissante comprenant au moins un hydroxystilbène.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même de différentes protéines extracellulaires parmi lesquelles figure notamment les fibres de collagène, l'élastine et différentes glycoprotéines. L'ensemble de ces composants extracellulaires est synthétisé par le fibroblaste. On trouve également dans le derme des leucocytes, des mastocytes ou encore des macrophages tissulaires. Enfin le derme contient des vaisseaux sanguins et des fibres nerveuses.

Le fibroblaste, de par son activité dans la synthèse des protéines extracellulaires matricielles (protéoglycannes, fibres de collagène et autres glycoprotéines de structure) est l'acteur principal de l'élaboration structurelle du derme.

Les fibres de collagène assurent la solidité du derme. Elles sont très résistantes mais sensibles à certaines enzymes appelées généralement collagénases. Dans le derme, les fibres de collagène sont constituées de fibrilles scellées les unes aux autres, formant ainsi plus de dix types de structures différentes. La structure du derme est en grande partie due à l'enchevêtrement des fibres de collagène tassées. Les fibres de collagène participent à la tonicité de la peau.
Les fibres de collagènes sont régulièrement renouvelées mais ce renouvellement diminue avec l'âge ce qui entraîne notamment un amincissement du derme. Il est également admis que des facteurs extrinsèques comme les rayons ultraviolets ou le tabac ont également un effet sur la peau et sur son taux de collagène.

Cependant, divers facteurs entraînent la dégradation du collagène, avec toutes les conséquences que l'on peut envisager sur la structure et/ou la fermeté de la peau et/ou des muqueuses.

Bien que très résistantes, les fibres de collagène sont sensibles à certaines enzymes appelées collagénases. Une dégradation des fibres de collagène entraîne l'apparence de peau molle et ridée que l'être humain, préférant l'apparence d'une peau lisse et tendue, cherche depuis toujours à combattre.

Les collagénases font partie d'une famille d'enzymes appelées métalloprotéinases (MMPs) qui sont elles-mêmes les membres d'une famille d'enzymes protéolytiques (endoprotéases) qui possèdent un atome de zinc coordonné à 3 résidus cystéine et une méthionine dans leur site actif et qui dégradent les composants macromoléculaires de la matrice extracellulaire et des lames basales à pH neutre (collagène, élastine, etc....). Très largement répandues dans le monde vivant, ces enzymes sont présentes, mais faiblement exprimées, dans des situations physiologiques normales comme la croissance des organes et le renouvellement des tissus.

La famille des métalloprotéinases est constituée de plusieurs groupes bien définis basés sur leurs ressemblances en terme de structure et de spécificité de substrat (voir Woessner J. F., Faseb Journal, vol. 5, 1991, 2145). Parmi ces groupes, on peut citer les collagénases destinées à dégrader les collagènes fibrillaires (MMP-1 ou collagénase interstitielle, MMP-8 ou collagénase de neutrophile, MMP-13 ou collagénase 3), les gélatinases qui dégradent le collagène de type IV ou toute forme de collagène dénaturé (MMP-2 ou gélatinase A (72 kDa), MMP-9 ou gélatinase B (92 kDa) ), les stromélysines (MMP-3) dont le large spectre d'activité s'adresse aux protéines de la matrice extracellulaire telles que les glycoprotéines (fibronectine, laminine), les protéoglycannes, etc., ou encore les métalloprotéinases membranaires.
L'exposition prolongée aux rayonnements ultraviolets, particulièrement aux rayonnements ultraviolets de type A et/ou B, a pour effet une stimulation de l'expression des collagénases, particulièrement de la MMP-1. C'est là une des composantes du vieillissement cutané photo-induit.

Par ailleurs, à la ménopause, les principales modifications concernant le derme sont une diminution du taux de collagène et de l'épaisseur dermique. Cela entraîne chez la femme ménopausée un amincissement de la peau. La femme ressent alors une sensation de "peau sèche" ou de peau qui tire et l'on constate une accentuation des fines rides et ridules de surface. La peau présente un aspect rugueux à la palpation. Enfin la peau présente une souplesse diminuée.
Il est démontré que les femmes perdent progressivement leur taux de collagène par an après la ménopause et que 30% du taux global est perdu dans les cinq premières années postménopausiques.

On comprend donc l'importance que revêt la présence de fibres de collagène dans la peau et l'importance qu'il existe à maintenir, voire renforcer, leur présence.

Il est donc important de pouvoir disposer de produits dont les effets visent à maintenir le taux de collagène dans la peau et lui maintenir une apparence lisse et tendue.

A cet égard la demanderesse a de manière surprenante et inattendue découvert que les hydroxystilbènes présentent la propriété de stimuler la synthèse du collagène et/ou de stimuler la prolifération des fibroblastes du derme et/ou d'inhiber l'expression des protéases de la matrice extracellulaire, particulièrement des métalloprotéinases et encore plus particulièrement de la métalloprotéinase de type 1.

Les hydroxystilbènes sont des composés répondant à la formule générale I : 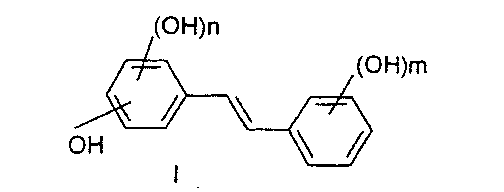 dans laquelle n est un nombre entier compris entre 0 et 4 inclusivement et m est un nombre entier compris entre 0 et 5 inclusivement. Ces composés peuvent être sous une forme Cis ou Trans.
Selon l'invention, le terme hydroxystilbène recouvre aussi bien les composés de formule I que leurs dérivés hydroxyalkylés.

Les hydroxystilbènes sont des composés que l'on retrouve à l'état naturel dans les végétaux de la classe des spermatophytes et particulièrement dans la vigne. On trouve de tels composés comme par exemple le resvératrol dans le raisin et dans le vin.

Dans l'art antérieur les hydroxystilbènes sont utilisés entre autres comme agents dépigmentants (JP87-192040), comme agents vasodilatateurs (EP 96-830517), comme agents antithrombiques (JP 05016413), dans le traitement de diverses affections cardio-vasculaires (CA 2187990), comme agents inhibiteurs de mutagenèse et de carcinogenèse (JP 06024967), ou encore décrits comme antioxydants.

Parmi ces composés le resvératrol (ou 3, 5, 4'-trihydroxystilbène) est particulièrement étudié pour les activités ci-dessus décrites principalement parce qu'il s'agit d'un composé naturel qui se retrouve dans la peau des grains de raisin et dans le vin. A cet égard on peut consulter la revue de Soleas et collaborateurs (Clinical Biochemistry, vol. 30, N°2, pp. 91-113, 1997) qui résume parfaitement l'état des connaissances concernant ce composé et les hydroxystilbènes.

Mais, la capacité des hydroxystilbènes à stimuler la synthèse du collagène et/ou la prolifération des fibroblastes du derme et/ou l'inhibition de l'expression des protéases de la matrice extracellulaire, n'a jamais été décrite à ce jour.

L'invention a donc pour objet l'utilisation, en tant que principe actif, dans ou pour la préparation d'une composition, d'une quantité efficace d'au moins un hydroxystilbène, cet hydroxystilbène ou la composition étant destinés à stimuler la synthèse du collagène et/ou la prolifération des fibroblastes du derme et/ou à inhiber l'expression des protéases de la matrice extracellulaire, particulièrement des métalloprotéinases et encore plus particulièrement de la métalloprotéinase de type 1.
Parmi les hydroxystilbènes, on peut citer les mono, di, tri, tétra, penta, hexa, hepta, octo, nonahydroxystilbènes, ou encore leurs dérivés hydroxyalkylés.

Selon l'invention les hydroxystilbènes peuvent être utilisés seuls ou en mélanges de toute nature et peuvent être d'origine naturelle ou synthétique.

Les hydroxystilbènes utilisables selon l'invention sont choisis parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2, 3',4,4',6-pentahydroxystilbène,
le 2,2',4,4',6,6'-hexahydroxystilbène.

Préférentiellement, on utilise selon l'invention du 3,4',5-trihydroxystilbène ou resvératrol.

Particulièrement l'hydroxystilbène ou la composition le contenant sont utilisés selon l'invention en application topique sur la peau.

On a vu précédemment que le collagène est impliqué dans la solidité du derme, donc dans la fermeté de la peau et/ou des muqueuses et que les fibroblastes sont responsables de la synthèse des protéines de la matrice extracellulaire du derme, notamment du collagène.

Ainsi, un des aspects de l'invention est donc de proposer l'utilisation en tant que principe actif dans ou pour la préparation d'une composition d'une quantité efficace d'au moins un hydroxystilbène, cet hydroxystilbène ou la composition étant destinés à traiter, de manière préventive ou curative, les signes cutanés du vieillissement, plus particulièrement à traiter, de manière préventive ou curative, la peau flasque et/ou ridée.

Un autre aspect de l'invention est de proposer l'utilisation en tant que principe actif dans ou pour la préparation d'une composition d'une quantité efficace d'au moins un hydroxystilbène, cet hydroxystilbène ou la composition étant destinés à stimuler le raffermissement de la peau.

Selon aussi un autre aspect, l'invention a pour objet l'utilisation en tant que principe actif dans ou pour la préparation d'une composition d'une quantité efficace d'au moins un hydroxystilbène, cet hydroxystilbène ou la composition étant destinés à favoriser le lissage de la peau et/ou à tendre la peau.

Selon encore un autre aspect, l'invention a pour objet l'utilisation en tant que principe actif dans ou pour la préparation d'une composition d'une quantité efficace d'au moins un hydroxystilbène, cet hydroxystilbène ou la composition étant destinés à combattre les effets cutanés de la ménopause, plus particulièrement les effets de la ménopause sur le collagène et/ou les fibroblastes.

La quantité d'hydroxystilbène utilisable selon l'invention dépend bien évidemment de l'effet recherché et doit être en une quantité efficace pour stimuler la synthèse du collagène et/ou la prolifération des fibroblastes du derme et/ou pour inhiber l'expression des protéases de la matrice extracellulaire.

A titre d'exemple la quantité d'hydroxystilbène utilisable selon l'invention peut aller par exemple de 0,001 % à 10 % et de préférence de 0,005 % à 5 % du poids total de la composition.

Par milieu cosmétiquement acceptable on entend un milieu compatible avec la peau, les muqueuses, les ongles, les cheveux.

La composition selon l'invention comprend bien évidemment un support cosmétiquement acceptable et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré.

L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxy-acides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.
Il est également possible d'utiliser en association avec l'hydroxystilbène utilisé selon l'invention des composés choisis parmi
- les hormones végétales ;
- des capteurs de radicaux OH, tels que le diméthylsulfoxyde ;
- des ouvreurs de canaux chlore ;
- des extraits de végétaux tels que ceux d'Iridacées, de Rosacées ou de soja, extraits pouvant alors contenir ou non des isoflavones ;
- des extraits de micro-organismes dont en particulier des extraits bactériens comme ceux de bactéries filamenteuses non photosynthétiques ;

A la liste ci-dessus, d'autres composés peuvent également être rajoutés, à savoir par exemple des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, des caroténoides, les acides eicosatétraénoïque et eicosatriénoïque ou leurs esters et amides, la vitamine D et ses dérivés.

Selon l'invention, on peut, entre autres, associer au moins un hydroxystilbène à d'autres agents actifs. Parmi ces agents actifs, on peut citer à titre d'exemple :
- les agents modulant la pigmentation cutanée tels que l'acide kojique ou l'hydroquinone ;
- les agents modulant l'adhésion bactérienne sur la peau et /ou les muqueuses tels que le miel, notamment le mile d'acacias et certains dérivés de sucres ;
- les agents kératolytiques tels que les acides alpha- et bêta-hydroxycarboxyliques ou bêta-cétocarboxyliques, leurs sels, amides ou esters et plus particulièrement les hydroxy-acides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antipelliculaires comme l'octopirox ou la pyrithione de zinc ;
- les antiacnéiques comme le peroxyde de benzoyle.
- des substances telles que les antagonistes de substance P, de CGRP ou de bradykinine ou les inhibiteurs de NO synthase ou encore les inhibiteurs de canaux sodiques, composés décrits comme étant actifs dans le traitement des peaux sensibles et comme présentant des effets anti-irritants, en particulier vis-à-vis de composés irritants éventuellement présents dans les compositions.

Comme actifs, on peut utiliser notamment les hydratants tels que les polyols (par exemple la glycérine), les vitamines (par exemple le D-panthénol), les agents apaisants (allantoïne, eau de bleuet), les filtres UVA et UVB, les agents matifiants (par exemple les polydiméthylorganosiloxanes partiellement réticulés vendus sous le nom KSG® par Shin Etsu) et leurs mélanges.

On peut aussi ajouter des actifs antirides, et notamment des produits tenseurs tels que les protéines végétales et leurs hydrolysats, en particulier l'extrait de protéines de soja vendue sous le nom d'Eleseryl® par la société LSN ou le dérivé d'avoine vendu sous la dénomination Reductine® par la société Silab.

La peau étant constituée de bien d'autres composants que le collagène et les fibroblastes, il s'avère intéressant, lorsque l'on utilise un hydroxystilbène selon l'invention, de favoriser en même temps la synthèse de ces autres composants comme par exemple les lipides et/ou de favoriser la prolifération d'autres composantes cellulaires comme par exemple les kératinocytes.

Ainsi, l'invention a pour objet une composition cosmétique comprenant dans un milieu cosmétiquement acceptable au moins un hydroxystilbène et au moins un autre produit stimulant la synthèse des lipides et/ou la prolifération des kératinocytes.

A cet égard on peut citer comme produit stimulant la synthèse des lipides les hormones végétales, comme les auxines, ou des composés d'origine végétale, comme l'acide cinnamique et comme produit stimulant la prolifération des kératinocytes des composés d'origine végétale, comme le phloroglucinol.

Ainsi, les compositions selon l'invention peuvent comprendre en plus de l'hydroxystilbène, de l'acide cinnamique ou ses dérivés et/ou une hormone végétale, particulièrement une auxine choisie parmi l'acide indolacétique (IAA), l'acide 4-chloroindole-3-acétique (4-Cl-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'idole acétaldéhyde et l'indole acétonitrile et/ou un composé végétal comme le phloroglucinol.

Bien entendu, les hydroxystilbènes peuvent être utilisés dans la préparation de compositions cosmétiques et/ou pharmaceutiques, particulièrement dermatologiques, destinées à stimuler la synthèse du collagène et/ou la prolifération des fibroblastes du derme.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral, sauf indications contraires.

### Exemple 1 : Etude de l'effet du resvératrol sur la synthèse du collagène.

L'étude est faite par mesure de l'incorporation de proline radioactive dans les protéines néosynthétisées par des cultures de fibroblastes dermiques humains normaux. Ces protéines néosynthétisées sont majoritairement des fibres de collagènes.

Les cultures de fibroblastes sont effectuées selon les méthodes classiques de culture cellulaire, à savoir en milieu MEM/M199 vendu par la société Gibco, en présence de bicarbonate de sodium (1,87 mg/ml) de L-glutamine (2 mM), de pénicilline (50 Ul/ml) et de 10% de sérum de veau foetal (Gibco).

Le test est effectué sur des cultures de cellules à 80% de confluence en plaque de 24 trous. Le resvératrol, à la concentration de 10⁻⁴M, est mis en contact avec les cellules pendant 48 heures. Le marquage à la proline tritiée (L-[2,3-³H]-proline vendue par Amersham, 33 µCi/ml) est effectué pendant 24 heures. Le taux de proline tritiée incorporée est mesuré en fin de test par précipitation acide des protéines sur filtres et comptage en scintillation liquide.

Les résultats sont évalués par rapport à un témoin constitué par des cellules n'ayant pas été traitées au resvératrol.

Un témoin positif (vitamine C à 20µg/ml) connue pour stimuler la synthèse du collagène est introduit dans le test comme référence.

Les résultats de ce test sont présentés dans le tableau suivant.

| Traitement | cpm | d.s; | % | p |
|---|---|---|---|---|
| Non traité | 22528 | 1918 | 100 | - |
| Resvératrol 1.25 µM | 27164 | 502 | 121 | <0.01 |
| Resvératrol 5.00 µM | 25769 | 1112 | 114 | <0.05 |
| Vitamine C (référence du test) | 36431 | 417 | 162 | <0.01 |
| cpm : coups par minutes. | | | | |
| d.s. : déviation standard | | | | |
| p : intervalle de confiance calculé selon la méthode de Dunett. | | | | |

Ces résultats montrent que le resvératrol stimule de façon significative l'incorporation de proline dans le collagène et donc qu'il active la néosynthèse de protéines, notamment de collagène.

### Exemple 2 : Etude de l'effet du resvératrol sur la prolifération des fibroblastes du derme.

L'étude est faite par mesure de l'incorporation de thymidine radioactive dans des cultures de fibroblastes dermiques humains normaux.
Les cultures de fibroblastes sont effectuées selon les méthodes classiques de culture cellulaire, à savoir en milieu MEM/M199 vendu par la société Gibco, en présence de bicarbonate de sodium (1,87 mg/ml) de L-glutamine (2 mM), de pénicilline (50 Ul/ml) et de 10% de sérum de veau foetal (Gibco).

Le test est effectué sur des cultures de cellules à 80% de confluence en plaque de 24 trous. Le resvératrol, à la concentration de 1,25 µM et 5 µM, est mis en contact avec les cellules pendant 48 heures. Le marquage à la thymidine tritiée ([méthyl-3H]thymidine vendue par la société Amersham, 82 Ci/mmole) est effectué pendant 24 heures.
Le taux de thymidine tritiée incorporée est mesuré en fin de test par précipitation acide des protéines sur filtres et comptage en scintillation liquide.

Les résultats sont évalués par rapport à un témoin constitué par des cellules n'ayant pas été traitées au resvératrol.

Un témoin positif (Sérum de veau foetal à 20%) connu pour stimuler la synthèse du collagène est introduit dans le test comme référence.

Les résultats de ce test sont présentés dans le tableau suivant.

| Traitement | cpm | d.s. | % | p |
|---|---|---|---|---|
| Non traité | 13649 | 1076 | 100 | - |
| Resveratrol 1.25 µM | 15055 | 831 | 110 | <0.05 |
| Resveratrol 5.00 µM | 19229 | 1407 | 141 | <0.01 |
| SVF 20 % (référence du test) | 17186 | 1426 | 126 | <0.05 |
| cpm : coups par minutes. | | | | |
| d.s. : déviation standard | | | | |
| p : intervalle de confiance calculé selon la méthode de Dunett. | | | | |

### Exemple 3 : Effet du resvératrol sur l'expression des collagénases.

L'effet du resvératrol sur la production de la collagénase interstitielle a été évalué dans un modèle de culture de cellules A2058 (issues de mélanomes humain: Templeton N.S. et al.1990; Cancer Res., 50: 5431-5431).
Les cellules sont incubés dans un milieu MEM sans rouge phénol contenant des acides aminés à la concentration de 2 mM, du pyruvate de sodium à la concentration de 1 mM et du sérum de veau charbonné à 10%. Elles sont ensuite mises en culture à la densité de 20000 cellules par puits dans les 24 puits de plaque multipuits.
Vingt quatre heures après la mise en culture, les cellules sont mises en contact avec le resvératrol. La production de la collagénase interstitielle est évaluée 96 heures plus tard dans le milieu de culture. Celle-ci est réalisée à l'aide d'un kit Elisa (Biotrack human MMP1; Amersham)
Le resvératrol est testé aux concentrations 2.10⁻⁷ M et 2.10⁻⁶M
Les résultats exprimés en pourcentage représentent la diminution de la production de la collagénase interstitielle par apport au contrôle, c'est à dire par apport à une culture effectuée dans les mêmes conditions en l'absence de resvératrol.

| | Resvératrol 0.2µM | Resvératrol 2µM |
|---|---|---|
| % d'inhibition | 14% | 57% |

Les résultats montrent que le resvératrol diminue la production de la collagénase interstielle par les cellules A 2058 et ceci de façon dose-dépendante.

### Exemple 4 : Exemples de compositions selon l'invention. Ces compositions sont obtenues par les techniques habituelles couramment utilisées en cosmétique ou en pharmacie.

| Composition 1 : crème de soin | |
|---|---|
| Cire d'abeille | 1,50 % |
| Huile d'amandes d'abricot | 13,00 % |
| Parfum | 0,40 % |
| Resvératrol | 1,00 % |
| Xanthane | 0,50 % |
| Cyclopentadiméthylsiloxane | 5,00 % |
| Mono-di-palmitostéarate de sucrose | 3,00 % |
| Sesquistéarate de méthylglucose | 3,00 % |
| Acide stéarique | 1,00 % |
| Alcool cétylique | 3,00 % |
| Conservateurs | 0,30 % |
| Eau déminéralisée stérilisée qsp | 100,00 % |

| Composition 2 : huile corporelle | |
|---|---|
| Huile de vaseline | 47,99 % |
| Huile d'amandes d'abricot | 6,00 % |
| Parfum | 1,00 % |
| Resvératrol | 0,50 % |
| Cyclopenta diméthylsiloxane | 45,00 % |

| Composition 3 : lait démaquillant | |
|---|---|
| Palmitate d'éthyl-2 hexyle | 10,50 % |
| Fraction liquide de beurre de karité | 16,50 % |
| Conservateurs | 0,30 % |
| Parfum | 0,15 % |
| Resvératrol | 0,10 % |
| Hydroxyde de sodium | 0,04 % |
| Polymère carboxyvinylique | 0,20 % |
| Eau déminéralisée stérilisée | 69,80 % |

## Revendications

1. Utilisation dans une composition cosmétique ou pour la préparation d'une composition cosmétique et/ou dermatologique en tant que principe actif d'une quantité efficace d'au moins un hydroxystilbène, cet hydroxystilbène ou la composition étant destinés à stimuler la synthèse de collagène.

2. Utilisation dans une composition cosmétique ou pour la préparation d'une composition cosmétique et/ou dermatologique en tant que principe actif d'une quantité efficace d'au moins un hydroxystilbène, cet hydroxystilbène ou la composition étant destinés à stimuler la prolifération des fibroblastes du derme.

3. Utilisation dans une composition cosmétique ou pour la préparation d'une composition cosmétique et/ou dermatologique en tant que principe actif d'une quantité efficace d'au moins un hydroxystilbène, cet hydroxystilbène ou la composition étant destinés à inhiber l'expression des protéases de la matrice extracellulaire.

4. Utilisation dans une composition cosmétique ou pour la préparation d'une composition cosmétique et/ou dermatologique en tant que principe actif d'une quantité efficace d'au moins un hydroxystilbène, cet hydroxystilbène ou la composition étant destinés à inhiber l'expression des métalloprotéinases.

5. Utilisation dans une composition cosmétique ou pour la préparation d'une composition cosmétique et/ou dermatologique en tant que principe actif d'une quantité efficace d'au moins un hydroxystilbène, cet hydroxystilbène ou la composition étant destinés à inhiber l'expression de la métalloprotéinase de type 1.

6. Utilisation selon l'une des revendications 1 à 5, **caractérisée par le fait que** l'hydroxystilbène ou la composition sont destinés à stimuler le raffermissement de la peau.

7. Utilisation selon l'une des revendications 1 à 5, **caractérisée par le fait que** l'hydroxystilbène ou la composition sont destinés à combattre les effets cutanés de la ménopause.

8. Utilisation selon l'une quelconque des revendications précédentes, **caractérisée par le fait que** l'hydroxystilbène ou la composition est utilisé en application topique sur la peau et/ou les muqueuses.

9. Utilisation selon l'une quelconque des revendications précédentes, **caractérisée par le fait que** l'hydroxystilbène parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2,3',4,4',6-pentahydroxystilbène,
le 2,2',4,4',6,6'-hexahydroxystilbène.

10. Utilisation selon l'une quelconque des revendications précédentes, **caractérisée par le fait que** l'hydroxystilbène est du 3,4',5-trihydroxystilbène ou resvératrol.

11. Utilisation selon l'une quelconque des revendications précédentes, **caractérisée par le fait que** l'hydroxystilbène est présent en une quantité allant de 0,001 % à 10% du poids total de la composition

12. Utilisation selon la revendication précédente, **caractérisée par le fait que** l'hydroxystilbène est présent en une quantité allant de 0,005% à 5% du poids total de la composition.

## Claims

1. Use, in a cosmetic composition or for the preparation of a cosmetic and/or dermatological composition, as active ingredient, of an effective quantity of at least one hydroxystilbene, this hydroxystilbene or the composition being intended to stimulate the synthesis of collagen.

2. Use, in a cosmetic composition or for the preparation of a cosmetic and/or dermatological composition, as active ingredient, of an effective quantity of at least one hydroxystilbene, this hydroxystilbene or the composition being intended to stimulate the proliferation of the fibroblasts of the dermis.

3. Use, in a cosmetic composition or for the preparation of a cosmetic and/or dermatological composition, as active ingredient, of an effective quantity of at least one hydroxystilbene, this hydroxystilbene or the composition being intended to inhibit the expression of proteases of the extracellular matrix.

4. Use, in a cosmetic composition or for the preparation of a cosmetic and/or dermatological composition, as active ingredient, of an effective quantity of at least one hydroxystilbene, this hydroxystilbene or the composition being intended to inhibit the expression of metalloproteinases.

5. Use, in a cosmetic composition or for the preparation of a cosmetic and/or dermatological composition, as active ingredient, of an effective quantity of at least one hydroxystilbene, this hydroxystilbene or the composition being intended to inhibit the expression of type 1 metalloproteinase.

6. Use according to one of Claims 1 to 5, **characterized in that** the hydroxystilbene or the composition are intended to stimulate the toning of the skin.

7. Use according to one of Claims 1 to 5, **characterized in that** the hydroxystilbene or the composition are intended to combat the cutaneous effects of the menopause.

8. Use, according to any one of the preceding claims, **characterized in that** the hydroxystilbene or the composition is used for topical application to the skin and/or mucous membranes.

9. Use according to any one of the preceding claims, **characterized in that** the hydroxystilbene is chosen from:
4'-hydroxystilbene,
2',4'-dihydroxystilbene,
3',4'-dihydroxystilbene,
4,4'-dihydroxystilbene,
2',4',4-trihydroxystilbene,
3',4',4-trihydroxystilbene,
2,4,4'-trihydroxystilbene,
3,4,4'-trihydroxystilbene,
3,4',5-trihydroxystilbene,
2',3,4-trihydroxystilbene,
2,3',4-trihydroxystilbene,
2',2,4'-trihydroxystilbene,
2,4,4',5-tetrahydroxystilbene,
2',3,4',5-tetrahydroxystilbene,
2,2',4,4'-tetrahydroxystilbene,
3,3',4',5-tetrahydroxystilbene,
2,3',4,4'-tetrahydroxystilbene,
3,3',4,4'-tetrahydroxystilbene,
3,3',4',5,5'-pentahydroxystilbene,
2,2',4,4',6-pentahydroxystilbene,
2,3',4,4',6-pentahydroxystilbene,
2,2',4,4',6,6'-hexahydroxystilbene.

10. Use according to any one of the preceding claims, **characterized in that** the hydroxystilbene is 3,4',5-trihydroxystilbene or resveratrol.

11. Use according to any one of the preceding claims, **characterized in that** the hydroxystilbene is present in a quantity ranging from 0.001% to 10% of the total weight of the composition.

12. Use according to the preceding claim, **characterized in that** the hydroxystilbene is present in a quantity ranging from 0.005% to 5% of the total weight of the composition.

## Patentansprüche

1. Verwendung mindestens eines Hydroxystilben in einer wirksamen Menge in einer kosmetischen Zusammensetzung oder zur Herstellung einer kosmetischen und/oder dermatologischen Zusammensetzung als Wirkstoff, wobei das Hydroxystilben oder die Zusammensetzung dazu vorgesehen sind, die Kollagensynthese zu stimulieren.

2. Verwendung mindestens eines Hydroxystilben in einer wirksamen Menge in einer kosmetischen Zusammensetzung oder zur Herstellung einer kosmetischen und/oder dermatologischen Zusammensetzung als Wirkstoff, wobei das Hydroxystilben oder die Zusammensetzung dazu vorgesehen sind, die Proliferation der Fibroblasten der Dermis zu stimulieren.

3. Verwendung mindestens eines Hydroxystilben in einer wirksamen Menge in einer kosmetischen Zusammensetzung oder zur Herstellung einer kosmetischen und/ oder dermatologischen Zusammensetzung als Wirkstoff, wobei das Hydroxystilben oder die Zusammensetzung dazu vorgesehen sind, die Expression von Proteasen der extrazellulären Matrix zu inhibieren.

4. Verwendung mindestens eines Hydroxystilben in einer wirksamen Menge in einer kosmetischen Zusammensetzung oder zur Herstellung einer kosmetischen und/oder dermatologischen Zusammensetzung als Wirkstoff, wobei das Hydroxystilben oder die Zusammensetzung dazu vorgesehen sind, die Expression von Metalloproteinasen zu inhibieren.

5. Verwendung mindestens eines Hydroxystilben in einer wirksamen Menge in einer kosmetischen Zusammensetzung oder zur Herstellung einer kosmetischen und/oder dermatologischen Zusammensetzung als Wirkstoff, wobei das Hydroxystilben oder die Zusammensetzung dazu vorgesehen sind, die Expression der Metalloproteinase vom Typ 1 zu inhibieren.

6. Verwendung nach einem der Ansprüche 1 bis 5, **dadurch gekennzeichnet, dass** das Hydroxystilben oder die Zusammensetzung dazu vorgesehen sind, die Straffung der Haut zu stimulieren.

7. Verwendung nach einem der Ansprüche 1 bis 5, **dadurch gekennzeichnet, dass** das Hydroxystilben oder die Zusammensetzung dazu vorgesehen sind, die Wirkungen der Menopause auf die Haut zu bekämpfen.

8. Verwendung nach einem der vorhergehenden Ansprüche, **dadurch gekennzeichnet, dass** das Hydroxystilben oder die Zusammensetzung topisch auf die Haut und/oder die Schleimhäute angewandet werden.

9. Verwendung nach einem der vorhergehenden Ansprüche, **dadurch gekennzeichnet, dass** das Hydroxystilben ausgewählt ist unter:
4'-Hydroxystilben,
2',4'-Dihydroxystilben,
3',4'-Dihydroxystilben,
4,4'-Dihydroxystilben,
2',4',4-Trihydroxystilben,
3',4',4-Trihydroxystilben,
2,4,4'-Trihydroxystilben,
3,4,4'-Trihydroxystilben,
3,4',5-Trihydroxystilben,
2',3,4-Trihydroxystilben,
2,3',4-Trihydroxystilben,
2',2,4'-Trihydroxystilben,
2,4,4',5-Tetrahydroxystilben,
2',3,4',5-Tetrahydroxystilben,
2,2',4,4'-Tetrahydroxystilben,
3,3',4',5-Tetrahydroxystilben,
2,3',4,4'-Tetrahydroxystilben,
3,3',4,4'-Tetrahydroxystilben,
3,3',4',5,5'-Pentahydroxystilben,
2,2',4,4',6-Pentahydroxystilben,
2,3',4,4',6-Pentahydroxystilben,
2,2',4,4',6,6'-Hexahydroxystilben.

10. Verwendung nach einem der vorhergehenden Ansprüche, **dadurch gekennzeichnet, dass** es sich bei dem Hydroxystilben um das 3,4',5-Trihydroxystilben oder Resveratrol handelt.

11. Verwendung nach einem der vorhergehenden Ansprüche, **dadurch gekennzeichnet, dass** das Hydroxystilben in einer Menge von 0,001 bis 10 % des Gesamtgewichts der Zusammensetzung vorliegt.

12. Verwendung nach dem vorhergehenden Anspruch, **dadurch gekennzeichnet, dass** das Hydroxystilben in einer Menge von 0,005 bis 5 % des Gesamtgewichts der Zusammensetzung vorliegt.
